# EUROPEAN PATENT SPECIFICATION

(11) **EP 4 408 275 B1**
(45) Date of publication and mention of the grant of the patent: **13.08.2025**
(21) Application number: 22813703.0
(22) Date of filing: 30.09.2022
(51) Int. Cl.: A61B 5/11, A61B 5/00

(54) **SYSTEM AND METHOD FOR MAKING INSOLES OPTIMIZING MOVEMENT AND POSITION POSTURAL MOTOR PATTERNS OF A PATIENT**
SYSTEM UND VERFAHREN ZUR HERSTELLUNG VON EINLEGESOHLEN ZUR OPTIMIERUNG VON BEWEGUNGS- UND POSITIONS- HALTUNGSMOTORMUSTERN EINES PATIENTEN
SYSTÈME ET PROCÉDÉ DE FABRICATION DE SEMELLES INTÉRIEURES OPTIMISANT LES SCHÉMAS MOTEURS POSTURAUX DE MOUVEMENT ET DE POSITION D'UN PATIENT

(30) Priority: 01.10.2021 EP 21425044
(43) Date of publication of application: 07.08.2024
(73) Proprietor: Human Motor Patterns S.r.l., 00159 Roma (IT)
(72) Inventor: MARESCA, Giovanni, 00159 Roma (IT)
(74) Representative: Studio Torta S.p.A.
(86) International application number: PCT/IB2022/059328
(87) International publication number: WO 2023/053076

(56) References cited:
- EP-B1- 2 399 513
- US-A1- 2010 154 252
- US-A1- 2015 164 377
- US-A1- 2017 258 390
- US-B1- 9 974 478
- MUNOZ-ORGANERO MARIO ET AL: "Identification of Walking Strategies of People With Osteoarthritis of the Knee Using Insole Pressure Sensors", IEEE SENSORS JOURNAL, IEEE, USA, vol. 17, no. 12, 15 June 2017 (2017-06-15), pages 3909 - 3920, XP011650000, ISSN: 1530-437X, [retrieved on 20170522], DOI: 10.1109/JSEN.2017.2696303

## Description

### TECHNICAL SECTOR OF THE INVENTION

The present invention relates, in general, to the field of physical and rehabilitation medicine and, more specifically, to a system and a related method for making insoles that optimise postural motor patterns of position and movement of a patient.

### STATE OF THE ART

As is well known, the insoles are customised orthesis that allow, in orthostasis and during walking, a foot strike as functional as possible for the person who uses it.

Typically, the insoles are prescribed by specialist doctors (usually physiatrists and orthopaedists, but often also recommended by diabetologists and neurologists) and are made by orthopaedic technicians using different methodologies and/or materials.

These orthesis can be prescribed, in Italy, at the expense of the National Health System (NHS) for children up to the age of 18 who need them or for persons suffering from specific conditions of recognized disability.

In addition, it is becoming increasingly common nowadays to purchase insoles even privately or through private insurance, especially in the world of sport, prescribed by a specialist doctor.

In this context, two of the biggest problems currently encountered in the rehabilitation sector are related to the fact that the techniques used are too often operator-dependent, but above all, that the results obtained from the use of ready-made insoles are rarely validated and measured.

The realization of the insoles is commonly carried out by sensing the setup of the feet in a standing position, usually by means of a cast or by two-dimensional scanning, without however any correction of the postural setup preparatory to the definitive detections for the realization of the orthosis.

More specifically, today there is no instrument that allows a team of healthcare professionals to carry out a series of interventions aimed at rebalancing the patient's postural setup, validated by subjective (perceived by the patient) and objective (scientifically detectable) assessments, preparatory for making customised insoles.

Moreover, to date, in most cases, the insoles are handmade and without a proper verification of their effectiveness.

In this regard, it is worth noting that numerous feedbacks from patients after the realization of the prescribed insoles, as currently realized, show functionally mediocre results in most cases.

In such a scenario, US 9 974 478 B1 discloses systems and methods for helping subjects improve safety and efficiency of their movements, particularly subjects who have suffered an injury or suffer from a movement or other such disorder. The system comprises sensor units which may comprise accelerometers and gyroscopes for recording movement data, for example, at least one sensor unit worn on each foot, thigh, on one finger and on the torso or trunk. The system further comprises a processor which feeds the measurement data into a trained algorithm loaded into the processor. The trained algorithm then uses the measured movement data to determine, detect or predict some movement impairment, unsafe or undesirable movement, unsafe or undesirable condition, or symptom of a movement disorder and outputs a signal corresponding to a cue or stimulus, and optionally to a treatment or therapy command and/or subject customized treatment which may then be displayed on a display device, or as input to control a treatment device such as an electric stimulator, automated medicine delivery or titration device, or the like.

More specifically, US 9 974 478 B1 relates to a system and method for monitoring a subject's movement to detect or predict unsafe, undesirable, or impaired movements, or symptoms of movement disorders, and also a system for providing possible treatment methods for such conditions.

Moreover, US 9 974 478 B1 further relates to a method and system of providing cues or stimuli to the subject when such unsafe or undesirable movements, instabilities or symptoms are detected or predicted.

More particularly, US 9 974 478 B1 relates to a subject-customized and adaptive movement recovery system, and method of providing therapy and training to improve functional motor recovery and safety of movement of a subject suffering from an injury or from movement disorder(s).

### OBJECT AND SUMMARY OF THE INVENTION

By virtue of the great experience accumulated over many years of professional activity carried out in the field of physical and rehabilitation medicine as a doctor specialising in physiatrics, the inventor noted that in this sector there is a strong need for a technical tool that acts as a technical-functional support for making insoles and that is easily implementable and repeatable, mainly for two purposes: to have a final product that is as effective as possible for the user and to minimize the dependence on the personal and subjective evaluations of the operators involved (e.g., specialist doctors, orthopaedic technicians, etc.).

Therefore, the Applicant felt the need to develop an innovative technical solution that allows a rehabilitation team to make new-generation insoles, regardless of the personal and subjective evaluations of the operators involved.

In the light of what has just been explained, an aim of the present invention is to provide an innovative technical solution that enables making new-generation customised insoles, regardless of the personal and subjective evaluations of the healthcare professionals involved, while supporting them in the various stages of the process of making such insoles, from sensing the foot imbalance to the packaging of the final product.

This and other aims are achieved by the present invention as it relates to a system for making optimised insoles, as defined in the appended Claims.

### BRIEF DESCRIPTION OF THE DRAWINGS

In order to better understand the present invention, some preferred embodiments, provided for merely exemplary and non-limiting purposes will now be shown with reference to the enclosed drawings (not in a scale), wherein:
- Figure 1 schematically shows a system for making insoles that optimise the postural motor patterns of position and movement of a patient according to an embodiment of the present invention; and
- Figures 2A, 2B, 2C and 2D schematically show an example of use of the system of Figure 1.

### DETAILED DESCRIPTION OF POSSIBLE EMBODIMENTS OF THE INVENTION

The following description is provided to enable a person skilled in the art to make and use the invention. Various modifications to the embodiments set forth will be immediately clear to the persons skilled in the art and the general principles herein disclosed may be applied to other embodiments and applications without, however, departing from the protection scope of the present invention as defined in the enclosed Claims.

Therefore, the present invention should not be understood as limited to the sole embodiments described and shown, but it must be given the widest scope of protection in accordance with the characteristics defined in the appended Claims.

The present invention concerns an innovative system for making insoles that optimise postural motor patterns of position and movement of a patient, implemented by the postural system in order to enable a subject to maintain an upright position and/or to walk and/or to run.

The assumption of what will be explained later considers the operation of the complex human postural system that is responsible for the activation of a series of motor patterns whose main purpose, in orthostasis and during the walk, is to contain the centre of gravity of the body in the support base (the only possibility that prevents the subject from falling).

These motor patterns depend on various factors but they must obligatorily be different if the distribution of loads and forces at the base is different: a different positioning, in fact, of the various anatomical components of the feet involves, in order for the postural system to pursue its main purpose (not to fall), the need to activate different motor patterns.

The applicant has named this system "Brain Motor Patterns Optimizer Device for making insoles" (BMPOD), which allows to make customised insoles designed by sensing the ideal postural setup of each person.

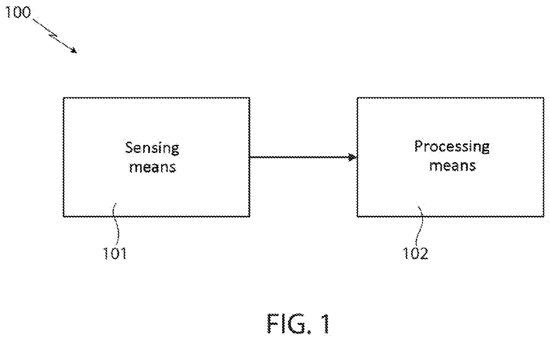

For a better understanding of the present invention, Figure 1 shows a high-level architecture (in particular by means of a block diagram) of a system (indicated as a whole with 100) for making insoles that optimise the postural motor patterns of position and movement of a patient according to an embodiment of the present invention (i.e., of the BMPOD).

In particular, as shown in Figure 1, the system 100 includes sensing means 101 and processing means 102.

The sensing means 101 are configured to perform a sensing function that includes:
- sensing quantities relating to a postural setup and to position and movement patterns of one or more predefined parts of a patient's body, preferably quantities relating to a postural setup and to position and movement patterns in orthostasis (i.e., in a standing position) and during walking, as well as the arrangement of the various components of the patient's feet; and
- generating, based on the quantities sensed, corresponding sensing data.

The sensing means 101 can be conveniently realized using sensing systems/devices/instruments, as well as sensors, of various types (e.g., cameras (for example infrared or other types), markers, position sensors, devices/sensors for sensing pressure points, centre of gravity, energy consumption or blood oxygenation, devices/systems for evaluating stability and ascending compensations and/or the global postural setup of the subject with recognition of the motor patterns implemented, optoelectronic systems, inertial sensors, pressure and/or deformation sensors, insoles with pressure sensors, sensorised platforms, baropodometric platforms, electromyographs, other systems for sensing muscle activities and static and dynamic postures such as for example continuous monitoring systems by mechanomyography, garments enabled in the nearby field with wireless and/or battery-free sensor network, other systems for analysis of static and dynamic postures, etc.).

The processing means 102 are connected (e.g., wired and/or wirelessly) to the sensing means 101 to acquire the sensing data generated by said sensing means 101 and are programmed to perform a processing and analysis function that includes:
- performing a predefined processing of the acquired sensing data by determining, based on the predefined processing performed, the postural setup and the position and movement patterns adopted by said predefined part(s) of the patient's body, preferably the postural setup and the position and movement patterns adopted in orthostasis and during walking and the arrangement of the various components of the patient's feet while these patterns are being adopted by the patient;
- analysing the postural setup and the position and movement patterns determined to detect one or more pathological patterns and one or more corresponding postural imbalances of said predefined part(s) of the patient's body, supported by imbalances in the setup of the patient's feet and/or of the lower limbs, conveniently, also identifying related distress areas; and
- determining, based on the pathological pattern(s) and on the corresponding postural imbalance(s) detected, one or more corresponding modifications to be made to the setup of the patient's feet and of the lower limbs, such as to allow the patient's postural system to modify the postural setup and/or the position and movement patterns adopted, so as to no longer adopt said pathological pattern(s) thanks to the correction of said corresponding postural imbalance(s).

Preferably, the processing means 102 are programmed to perform the processing and analysis function by:
- determining (i.e., generating), based on the acquired sensing data, multidimensional digital models relating to (conveniently representative of) said postural setup and said position and movement patterns adopted by said predefined part(s) of the patient's body in orthostasis and during walking; and
- detecting the pathological pattern(s) and the corresponding postural imbalance(s) and determining the corresponding modification(s) to be made based on the determined multidimensional digital models.

The system 100 is designed to be iteratively operated such that with each iteration:
- the sensing means 101 and the processing means 102 perform, respectively, the sensing function and the processing and analysis function after the modification(s) determined by the processing means 102 at the immediately preceding iteration has/have been made to the setup of the feet of the patient, resulting in a modification of the motor patterns of position and movement of the patient in orthostasis and during walking and/or running; and
- the processing means 102 determine one or more new modifications to be made to the setup of the feet in order to achieve an ideal postural setup of the predefined part(s) of the patient's body, preferably until reaching a global ideal postural setup in both bipodalic and monopodalic strike, which will be found with a very precise foot strike.

The system 100 is designed to no longer be operated to perform further iterations when the processing means 102 determines (at the last iteration of operation of said system 100) the achievement of the ideal overall postural asset by the predefined part(s) of the patient's body, which will correspond to a setup of the various anatomical components of the feet that is different from that sensed at the first iteration by the system 100.

In addition, the processing means 102 are also programmed to, once the ideal postural setup has been reached, generate, based on said ideal postural setup, data/information (conveniently in terms of 3D setup to be made, material(s) to be used, thickness(es) to be made, etc.) for making insoles such as to allow, when they are used by the patient, the predefined part(s) of the patient's body to adopt said ideal postural setup.

Preferably, the processing means 102 are configured to implement one or more predefined artificial intelligence technologies, conveniently one or more machine learning techniques and/or one or more neural networks, to:
- perform the processing and analysis function;
- determine the achievement of the ideal global postural setup and the precise positioning of the various parts of the feet that involved;
- generate the data/information for making the insoles.

The processing means 102 may be conveniently implemented according to different architectural paradigms, e.g., they may be implemented with a centralised (e.g., by means of a server) or distributed (e.g., by means of a cloud computing system) architecture, and may be connected to the sensing means 101 in a wired and/or wireless manner, e.g., they may also be remotely connected via one or more wireless communication technologies (e.g., 3G, 4G, 5G mobile phone technologies, Wi-Fi technology, etc.).

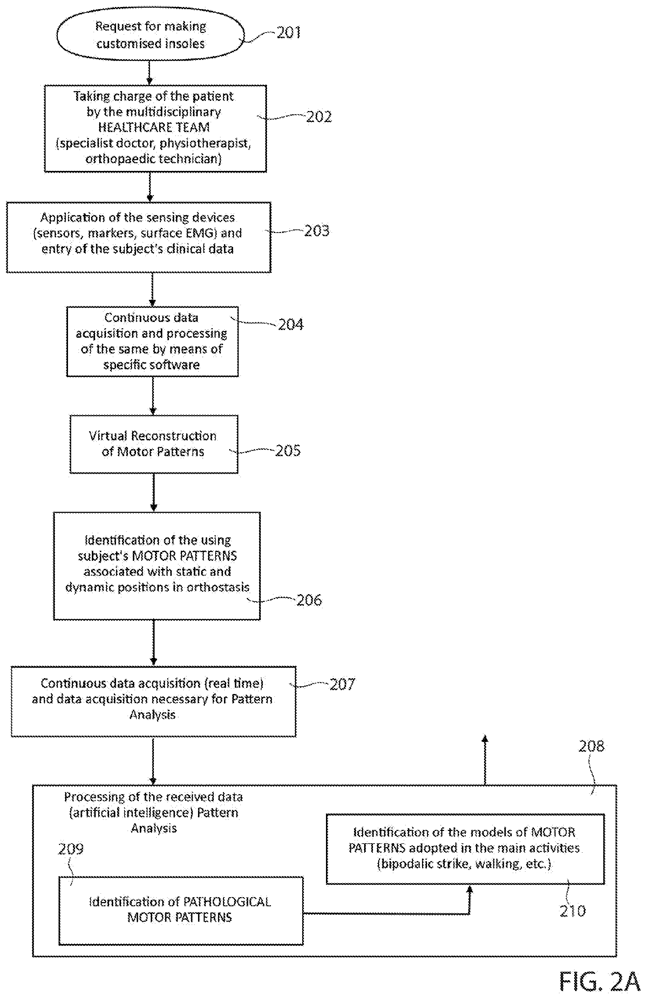

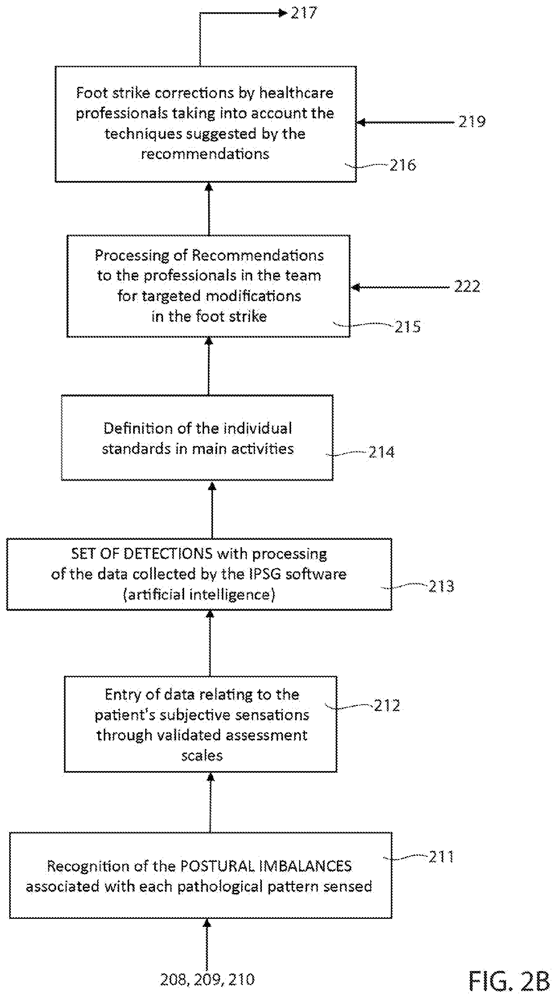

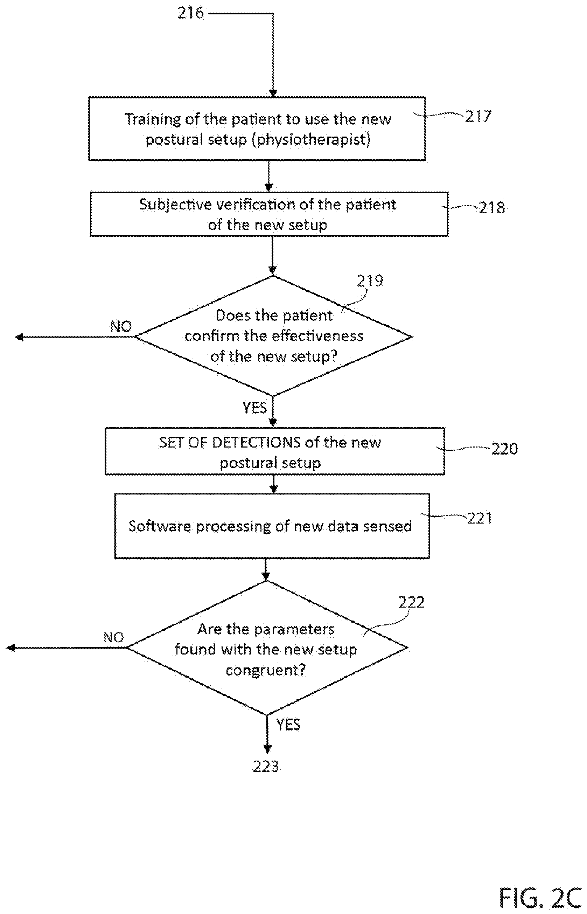

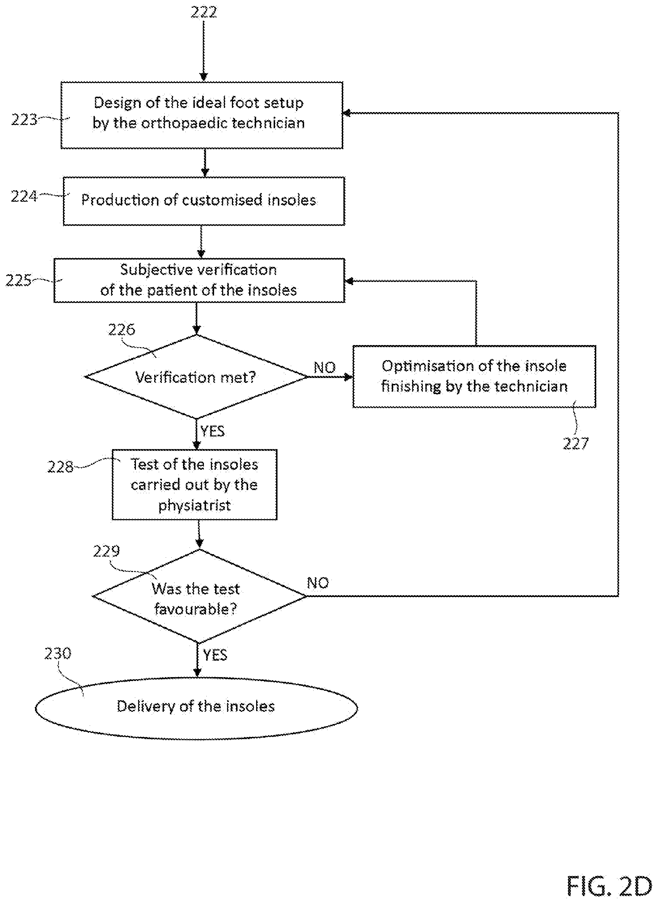

Figures 2A, 2B, 2C and 2D show (particularly by means of a flowchart) an example of use of the system 100 (i.e., the BMPOD) .

In particular, the steps of use of the system 100 (i.e. of the BMPOD) shown in Figures 2A-2D (and progressively numbered from 201 to 230) are absolutely self-explanatory and immediately understandable by any technician in the field, so they will not be described in detail here.

With the system 100 (i.e., the BMPOD), healthcare professionals can make a new generation of insoles, which allow that optimise the placement of the feet in standing position and during walking, eliminating (or, in any case, significantly reducing) a physical handicap in individuals who present postural imbalances accompanied and sustained by pathological motor patterns |caused by an incorrect foot strike, resulting in upward compensations. The final result, represented by an innovative insole that is the result of an evaluation of the static and dynamic postures in both bipodalic and monopodalic strike, of the recognition of imbalances of the foot strike that involve pastological patterns, followed by a correction of the same, improves the quality of life of these subjects and prevents the onset of further disabilities and consequent handicaps.

The insoles made with the system 100 (i.e., the BMPOD) allow the postural system to adopt more ergonomically effective motor patterns, in orthostasis and during walking and/or running, which are compared with the patterns used by the subject in the absence of orthesis or with ready-made insoles.

As explained above, the system 100 (i.e., the BMPOD) is made by hardware components such as sensing systems 101 (e.g., sensors, insoles, electromyographs, etc.) associated with one or more appropriate software programs (e.g., a software program/algorithm/module named by the Applicant "Ideal Patterns Search Guide" - IPSG) for the processing of data, sensed multidimensionally, in order to highlight the various motor patterns adopted by the subject as a consequence of that specific foot strike.

The system 100 (i.e., the BMPOD) also comprises software systems, also "embedded" if necessary, which are specifically made, integrated and calibrated exclusively for the application and functional purposes of said system 100 (i.e., of the BMPOD).

Thanks to these software components, the system 100 (i.e., the BMPOD) identifies the postural dysfunctions of each patient and the related pathological patterns adopted by him; moreover, once all the data acquired has been processed, it provides to suggest to the healthcare professionals involved the specific corrections of the foot setup; once the effectiveness of these corrections made by the operators has been verified by the system 100, the competent orthopaedic technician is allowed to elaborate the characteristics of the insoles, through the production of a three-dimensional (3D) digital project of the same, specific for each patient analysed, obtained from the three-dimensional (3D) detection of the ideal foot setup (i.e., the one resulted as the most effective for a correct functioning of the postural system).

The end result is to enable each individual subject to adopt more advantageous and effective motor patterns.

The use of the system 100 (i.e., the BMPOD) is intended for the exclusive use of a multidisciplinary team (which may be, for example, composed of doctor specialising in physiatrics, a physiotherapist and an orthopaedic technician). The team uploads the clinical information referring to the patient into the system 100 (i.e., into the BMPOD); the system 100 (i.e., the BMPOD) integrates these data with those coming from sensing systems (sensors, EMG, etc.) and, through its analysis algorithms (preferably through artificial intelligence systems/techniques/technologies), processes all the information acquired from the different sources, producing as output also the specific instructions to implement an articular and functional rebalancing that optimises the foot strike through a rehabilitation intervention.

In light of what has been explained above, the system 100 (i.e., the BMPOD) makes it possible to make new-generation customised insoles designed by sensing the ideal posture in monopodalic and bipodalic strike.

The system 100 (i.e., the BMPOD) can be conveniently used through the use of the following professional roles:
- doctor specialising in physiatrics;
- physiotherapist;
- orthopaedic technician.

The system 100 (i.e., the BMPOD) allows healthcare professionals to make insoles by carrying out foot detections in orthostasis with a different setup than classic detections. Therefore, an innovative insole will be made based on the setup obtained after the global functional rebalancing (muscle and joint) through corrections of the foot strike.

Thanks to the use of the present invention, it is possible to obtain customised insoles that accompany, with load, forefoot, midfoot and backfoot towards an optimal setup, allowing users to adopt more effective motor patterns in postural terms.

Foot measurements can be conveniently carried out through various methodologies, including three-dimensional (3D) scanning of the feet in orthostasis and, once the most effective corrections have been established, carried out and validated, new "ideal setup" measurements are carried out that allow the identification, choice and subsequent packaging of the customised insoles that optimise the position and movement postural motor patterns (preferably by 3D printing, in particular in the case where the setup is also sensed in 3D).

These insoles are made taking into account the static and dynamic neuromuscular activation patterns that are automatically activated by the postural system and are different if the distribution of loads and forces at the base is different.

Therefore, as also described above, the system 100 (i.e., the BMPOD), in order to make correct and functional insoles, conveniently includes some software subsystems, if necessary also "embedded", called IPSG ("Ideal Patterns Search Guide"), and a series of sensing systems 101 suitable to acquire the necessary information (e.g., sensors, surface electromyographs, etc.) for making the insoles, in addition to providing information as output to support the team's decisions regarding the rehabilitation interventions suitable for each specific case.

The IPSG software takes into account the information provided by the specialist doctor, the objective information from the sensing tools 101 and the subjective information of the patient (for example on the presence of pain, feeling of fatigue, instability, etc.) obtained by compiling assessment scales by the patient himself. This software allows to healthcare professionals (specialist doctor, physiotherapist and orthopaedic technician), by executing specific corrections suggested by the system 100 to the operators involved and verification of the same, to determine the ideal setup of the feet that corresponds to the one which allows the postural system of the subject in question to activate biomechanically more advantageous patterns, in both monopodalic and bipodalic strike.

Therefore, the software supports the realization and subsequent finding of the ideal positioning of the various anatomical components in orthostasis, including the feet, with the possibility for the orthopedic technician to design insoles on the new setup found, through various techniques (3D scans, casts, positive-negative cast, 3D printing, etc.).

Another important factor is that with the system 100 (i.e., the BMPOD), in the case of the presence of postural imbalances, it is possible to prevent damage from "ascending compensation" caused by "compulsory pathological patterns" (i.e., forced by the postural imbalances present, due to alterations in the physiological spatial relationships between the anatomical structures).

Once the orthesis (i.e., the new-generation insoles) have been made based on the ideal postural setup of an individual, the latter's postural system will benefit from a more suitable, therefore more effective and performing foot strike.

As also explained above, the system 100 (i.e., the BMPOD) is intended for the management of the problems deriving from postural dysfunctions caused by pathological motor patterns triggered by a human being in orthostasis, in both monopodalic and bipodalic strike, supported by imbalances of the foot strike.

The software component of the system 100 (i.e., of the BMPOD) has been designed in order to collect and process information from high-precision sensing systems 101, as well as from data provided directly by the specialist doctor or by the patient (in the latter case through validated evaluation scales). The processing of the data received is aimed at providing professionals with indications for corrections of the foot strike in order to improve the overall postural setup of the involved subject. The corrections are made using rehabilitation techniques aimed at optimising the foot strike so as to provide the postural system with the possibility of using, for that specific subject, motor patterns that are more functional than those previously sensed.

The software is conveniently designed with an open architecture so that it is not limited to receiving information from specific systems, being instead structured so that it can be easily interfaced with any sensing system 101 (and related information provided).

The IPSG software preferably includes two main components.

The former is a central logical core that implements the rules according to which the data from the detections are processed, resulting in a series of recommendations. The latter contain suggestions for specific interventions to be carried out on specific body districts, aimed at correcting the postural dysfunctions sensed. These interventions, by modifying the foot strike, allow the user to use more functional motor patterns and are carried out by the various professionals involved, depending on their specific skills, such as the physiotherapist or the orthopaedic technician.

The core team is also responsible for the collection of other data, e.g. those reported by the patient, as well as those noted and entered by the healthcare professionals, both concerning the clinical characteristics of the subject evaluated and, at a later stage, the performance or non-performance by the operator of the various recommendations produced.

Once it has been ascertained which recommendations have been made, a repetition of the detections is required, and the new values are compared with the previous ones, allowing the results produced on the subject by the recommendations made to be assessed using specific algorithms.

Artificial intelligence, machine learning techniques and neural networks are some of the possible methodologies/techniques/technologies conveniently exploited by the present invention.

The second of the two main components is a subsystem dedicated to interfacing the aforesaid central core with the sources of the data resulting from the various types of detection carried out on the subject (for example, physical ones by means of sensors that can also be worn, other sensing systems, information entered by healthcare professionals, etc.).

These sources can be of any kind, e.g. sensors applied to the subject, measurements taken by the operator, the operator's answers to questions submitted to him by the system, analysis of three-dimensional (3D) images, etc.

Preferably, the system 100 (i.e., the BMPOD) is able to interface very easily with any type of source for sensing both of objective and subjective data.

This minimises the effort required to adapt the system 100 (i.e., the BMPOD) to new sensing sources, ensuring that only the implementation of an adapter for the new source is required in a completely transparent manner with respect to the implementation of the algorithms contained in the central logical core.

The system 100 (i.e., the BMPOD) can be made both for use by operators via a web interface, and as a normal software application installed on local hardware systems.

The hardware components of the system 100 (i.e., of the BMPOD) interact with the software components and are used to carry out objective detections that allow the software to carry out a "motor patterns analysis".

The system 100 (i.e., the BMPOD) can conveniently use devices of various kinds (e.g., sensors for sensing the positions of points of the human body in space), based on different technologies (e.g., sensors based on NFC technology - acronym for "Near-field Communication"), applied, depending on the points to be examined and the type of sensor, directly on the skin or through clothing specifically provided for this purpose. Examples of sensing means 101 usable by the system 100 (i.e., by the BMPOD) are: cameras (e.g., either infrared or other types), markers, optoelectronic systems, inertial sensors, pressure and/or strain sensors, insoles with pressure sensors, sensorised platforms, baropodometric platforms, wired or wireless surface electromyographs, other systems for sensing muscle activities and static and dynamic postures such as continuous monitoring systems by mechanomyography, garments enabled in the nearby field with wireless and/or battery-free sensor network, other systems for analysing static and dynamic postures, etc.

The final products (i.e. insoles made by means of the present invention) are also designed with geometry perfectly compatible for each type of footwear suitable for the anthropometric characteristics of the subject involved.

All this allows, therefore, to obtain an ideal placement that takes into account not only the distribution of the loads, but also all the static and dynamic ascending balances related to it and implemented by neuro-muscular patterns whose sole purpose, in orthostasis and during the walk, is to contain the centre of gravity of the body in the placement base.

One of the advantages of the present invention is represented by the fact that it gives clinicians the possibility to also evaluate the effectiveness of the corrections made to the pathological setup detected, as well as the type of material (or materials) that is most suitable (or that are most suitable) for the case before the creation of the final insoles. In fact, it is possible to verify, through a comparison with one or more "status quo ante", the response of the adaptation of the subject or the subjective perceptions of the latter, adding important elements for the evaluation of the changes made, the choice of the material(s), the thickness(es) of the insoles and numerous other information.

The insoles made thanks to the present invention make it possible to improve balance, gait pattern, performance, foot strike load distribution, as well as venous and lymphatic return; furthermore, they make it possible to reduce the risk of accidental falls, energy expenditure, the incidence of distortive trauma in ankle inversion, ascending compensations from pathological patterns, joint and/or tendon overloads, dysfunctions of the spine.

The use of the system 100 (i.e., of the BMPOD) by healthcare personnel provides for the adoption, for the best use of the present invention, of spaces and means suitable for the purpose; a special station can be conveniently used to carry out the detections useful for the design and packaging of the insoles, which for example:
- can be based on traditional technologies and systems (for example footprints with phenolic foam and negative models with plaster cast, or systems with two-dimensional scanning) ;
- can preferably be configured to implement three-dimensional (3D) measurements with automatic scanning of the feet in orthostasis.

The system 100 (i.e., the BMPOD) provides useful information and indications to:
- quantitatively determine the target parameters, allowing the typological and quantitative identification of any imbalances and related pathological motor patterns;
- propose to healthcare professionals any corrections to be made, taking into account the motor patterns used and the individual characteristics of the subject previously requested, preferably through the use of an artificial intelligence system.

Once the necessary corrections have been made to the foot strike by the physiotherapist and/or orthopaedic technician and/or physiatrist and the functional response in a static and dynamic way has been evaluated (also through an innovative motor pattern analysis model), as well as the subjective perception of the patient, the type of material/s most functional to the case has been established, the system 100 (i.e., the BMPOD) carries out the final detection of the ideal setup of the foot that allows making a customised insole model that is as functional as possible for the individual, improving his performance, reducing or eliminating ascending compensations and allowing the elimination of automatic pathological patterns of position and movement in orthostasis and during walking and at the same time replacing them by creating more performing position and movement patterns.

The following table briefly indicates the market needs and/or use functions required by the market and, for each of these needs/use functions, compares the relative innovative characteristics of the present invention with respect to the traditional solutions and indicates the relative advantages of the present invention.

| **Market needs - Use functions required by the market** | **Innovative features of the present invention** | **Traditional solutions** | **Advantages of the present invention** |
|---|---|---|---|
| **Management of handicaps resulting from imbalances in the foot strike through a rehabilitation project** | Integration and combination of technologies, even of a known type, used, however, in an innovative way. Production of insoles through work carried out in a multidisciplinary team by optimising motor patterns using specific software. | Rehabilitation project, not always carried out with the intervention of several professionals, in which the packaging of insoles can also be inserted. The latter are, however, simply prescribed by the specialist doctor and made by the orthopaedic technician separately. | Possibility of verification and objective measurement of the results obtained. Improved treatment effectiveness, greater efficiency in preventing further handicaps, lower costs to achieve the final result, i.e. improving the quality of life of the subjects by eliminating or reducing handicaps and preventing disabilities. |
| **Choice of insole materials** | Search for materials best suited to the purpose and combination of several materials. | Use of standard materials without the possibility of evaluating whether the material chosen is the most suitable to solve the specific problem. | Packaging of insoles based on a choice of material according to the subjective and objective evaluation of the clinical response. |
| **Insole production** | Production of customised insoles after correction of the setup and subjective and objective verifications of the effectiveness of the new setup, as well as with customised materials in combination with each other. | Realization carried out by an orthopaedic technician on a cast or other type of detection in his charge without previous targeted changes in the postural setup. | Creation of a three-dimensional (3D) model of the insole made based on each person's ideal postural asset (i.e. the one that allows the postural system to adopt more ergonomically effective motor patterns, in orthostasis and during walking). |

In addition, the use of the present invention for making orthotics to support sporting activities also makes it possible to achieve a significant reduction in the risk of injury and a significant improvement in performance (e.g., in terms of stability, balance, energy expenditure, etc.).

As explained above, the present invention uses one or more processing system(s)/device(s) 102 that is(are) suitably programmed by means of one or more software programmes for:
- the management, processing and customisation of the data provided by one or more external detectors 101 (e.g., one or more devices or sensors that preferably also include a 3D scanner);
- the search for the ideal setup, which will be established based on subjective and objective evaluations;
- the choice (i.e. the selection) of the correction(s) to be made to the foot setup based on the motor patterns adopted for each specific setup;
- the choice (i.e. the selection) of one or more materials to make the various parts that make up the orthosis;
- the design and production of the ideal insoles, preferably through 3D printing.

The present invention can conveniently use an innovative station configured to sense and reconstruct in 3D the feet of the feet when standing.

In this regard, it should be noted that the setup of the feet is different depending on the types of material used for the packaging of the insoles and that part of the feet may end up more or less below the placement plane.

Therefore, it may be difficult to obtain an exact representation of the part of the feet that interfaces with the material that will constitute the insoles in the event that 3D scanning is limited. To overcome this problem, elastic fabrics with sensors can be conveniently used for the 3D reconstruction of the sole of the feet in that state or, alternatively, transparent materials with planes of different resistance, equivalent to that of the materials that will constitute the insoles, which allow a complete scanning in orthostasis of the feet.

With regard to the devices/sensors used by the present invention for 3D sensing of the setup of a patient's foot or feet, one or more of the following can also be conveniently employed: position sensors, electromyographs, markers, devices/sensors for sensing pressure points, barycentre, energy consumption or blood oxygenation, evaluation of stability and ascending compensation, global postural setup of the subject with recognition of the implemented motor patterns, etc.

Moreover, the aforesaid processing system(s) and/or device(s) 102 may be conveniently programmed also based on subjective parameters (e.g., obtained based on rating scales on quality of life, pain, risk of falls, feeling of fatigue, etc.) .

In the light of what has been described above, the system according to the present invention (i.e., the BMPOD) allows, after measuring objective-parameters, a multidisciplinary rehabilitation team to produce new-generation insoles, which restore the correct value of said parameters; subsequent monitoring and measurements also allow to evaluate the expected effectiveness and to progressively modify, if necessary, the geometry of the insoles.

From the foregoing description, the multiple innovative features and the innumerable technical advantages of the present invention are immediately apparent to one skilled in the art.

In conclusion, it is important to note that, while the above-described invention refers in particular to very specific embodiments, it must not be intended as limited to such embodiments, including within its scope all the variants, modifications or simplifications covered by the enclosed Claims.

## Claims

1. System (100) for making optimised insoles, comprising sensing means (101) and processing means (102);
wherein the sensing means (101) are configured to perform a sensing function which includes:
• sensing quantities relating to
- a postural setup and position and movement patterns of one or more predefined parts of the body of a patient in orthostasis and during walking, and
- an arrangement of the various components of the feet of the patient in orthostasis and during walking; and
• generating, based on the quantities sensed, corresponding sensing data;
wherein the processing means (102) are configured to acquire from the sensing means (101) the sensing data generated by said sensing means (101) and are programmed to perform a processing and analysis function including:
• performing a predefined processing of the acquired sensing data by determining, based on the predefined processing performed,
- the postural setup and the position and movement patterns adopted by said predefined part(s) of the body of the patient in orthostasis and during walking and
- the arrangement of the various components of the feet of the patient while said position and movement patterns are being adopted by the patient;
• analysing the postural setup and the position and movement patterns determined to detect one or more pathological patterns and one or more corresponding postural imbalances of said predefined part(s) of the patient's body; and
• determining, based on the pathological pattern(s) and the corresponding postural imbalance (s) detected, one or more corresponding changes to be made to the setup of the patient's feet and of the lower limbs, such as to allow to modify the postural setup and/or the position and movement patterns adopted by said predefined part(s) of the patient's body, so as to no longer adopt said pathological scheme(s) thanks to the correction of said corresponding postural imbalance(s).

2. The system of Claim 1, wherein the processing means (102) are programmed to perform the processing and analysis function by:
• determining, based on the acquired sensing data, multidimensional digital models relating to said postural setup and said position and movement patterns adopted by said predefined part(s) of the patient's body in orthostasis and during walking; and
• detecting the pathological pattern(s) and the corresponding postural imbalance(s) and determining the corresponding modification(s) to be made based on the determined multidimensional digital models.

3. The system according to Claim 1 or 2, wherein said system (100) is designed to be iteratively operated such that with each iteration:
• the sensing means (101) and the processing means (102) perform, respectively, the sensing function and the processing and analysis function after the change(s) determined by the processing means (102) at the immediately preceding iteration has/have been made to the setup of the patient's feet so as to determine a consequent modification of the postural setup and of the position and movement patterns of the predefined part(s) of the patient's body in orthostasis and during walking; and
• the processing means (102) determines one or more new modifications to be made to the setup of the feet in order to achieve an ideal postural setup of the predefined part(s) of the patient's body.

4. The system of Claim 3, wherein said system (100) is designed to no longer be operated to perform further iterations when the processing means (102) determines that the ideal postural setup has been reached by the predefined part(s) of the patient's body.

5. The system of Claim 4, wherein the processing means (102) is also programmed to, once the ideal postural setup has been reached, generate, based on said ideal postural setup, data/information for making insoles such as to allow, when used by the patient, the predefined part(s) of the patient's body to adopt said ideal postural setup.

6. The system of Claim 5, wherein the processing means (102) is configured to implement one or more predefined artificial intelligence technologies to:
• perform the processing and analysis function;
• determine that the ideal postural setup has been reached;
• generate the data/information for making the insoles.

7. The system of Claim 6, wherein said predefined artificial intelligence technology(s) includes one or more machine learning techniques and/or one or more neural networks.

8. Method for making optimised insoles, comprising using the system (100) as claimed in any preceding Claim to make insoles for a patient such as to allow to the latter to adopt an ideal postural setup.

## Patentansprüche

1. System (100) zur Herstellung optimierter Einlegesohlen, umfassend Erfassungsmittel (101) und Verarbeitungsmittel (102);
wobei die Erfassungsmittel (101) so konfiguriert sind, dass sie eine Erfassungsfunktion ausführen, die Folgendes einschließt:
• Erfassen von Größen in Bezug auf
- eine Haltungsaufstellung und Positions- und Bewegungsmuster eines oder mehrerer vordefinierter Körperteile eines/einer Patienten/Patientin in Orthostase und beim Gehen und
- eine Anordnung der verschiedenen Komponenten der Füße des/der Patienten/Patientin in Orthostase und beim Gehen; und
• Erzeugen entsprechender Erfassungsdaten auf der Grundlage der erfassten Größen;
wobei die Verarbeitungsmittel (102) so konfiguriert sind, dass sie von den Erfassungsmitteln (101) die von den Erfassungsmitteln (101) erzeugten Erfassungsdaten erwerben und so programmiert sind, dass sie eine Verarbeitungs- und Analysefunktion ausführen, die Folgendes einschließt:
• Durchführen einer vordefinierten Verarbeitung der erworbenen Erfassungsdaten durch Bestimmen, auf der Grundlage der durchgeführten vordefinierten Verarbeitung,
- der Haltungsaufstellung und der Positions- und Bewegungsmuster, die von dem/den vordefinierten Körperteil(en) des/der Patienten/Patientin in der Orthostase und beim Gehen eingenommen werden, und
- der Anordnung der verschiedenen Komponenten der Füße des/der Patienten/Patientin, während diese Positions- und Bewegungsmuster vom/von der Patienten/Patientin eingenommen werden;
• Analysieren der Haltungsaufstellung und der Positions- und Bewegungsmuster, die bestimmt wurden, um ein oder mehrere pathologische Muster und ein oder mehrere entsprechende Haltungsungleichgewichte des/der vordefinierten Körperteile(s) des/der Patienten/Patientin zu erkennen; und
• Bestimmen, auf der Grundlage des/der erkannten pathologischen Muster(s) und des/der entsprechenden Haltungsungleichgewichts(e), einer oder mehrerer entsprechender Änderungen, die an der Aufstellung der Füße des/der Patienten/Patientin und der unteren Gliedmaßen vorgenommen werden müssen, um es zu ermöglichen, die Haltungsaufstellung und/oder die Positions- und Bewegungsmuster, die von dem/den vordefinierten Körperteil(en) des/der Patienten/Patientin eingenommen werden, zu modifizieren, um das/die pathologische(n) Schema(Schemata) dank der Korrektur des/der entsprechenden Haltungsungleichgewichts(e) nicht mehr einzunehmen.

2. System nach Anspruch 1, wobei die Verarbeitungsmittel (102) programmiert sind, um die Verarbeitungs- und Analysefunktion auszuführen durch:
• Bestimmen, auf der Grundlage der erworbenen Erfassungsdaten, mehrdimensionaler digitaler Modelle in Bezug auf die Haltungsaufstellung und die Positions- und Bewegungsmuster, die von dem/den vordefinierten Körperteil(en) des/der Patienten/Patientin in Orthostase und während des Gehens eingenommen werden; und
• Erkennen des/der pathologischen Muster(s) und des/der entsprechenden Haltungsungleichgewichts(e) und Bestimmen der entsprechenden vorzunehmenden Modifikation(en) auf der Grundlage der bestimmten mehrdimensionalen digitalen Modelle.

3. System nach Anspruch 1 oder 2, wobei das System (100) so konfiguriert ist, dass es iterativ betrieben wird, so dass bei jeder Iteration:
- die Erfassungsmittel (101) und die Verarbeitungsmittel (102) die Erfassungsfunktion bzw. die Verarbeitungs- und Analysefunktion ausführen, nachdem die von der Verarbeitungsmittel (102) bei der unmittelbar vorhergehenden Iteration bestimmte(n) Änderung(en) an der Aufstellung der Füße des/der Patienten/Patientin vorgenommen wurde(n), um eine konsequente Modifikation der Haltungsaufstellung und der Position und der Bewegungsmuster des/der vordefinierten Teils/Teile des Körpers des/der Patienten/Patientin in Orthostase und beim Gehen zu bestimmen; und
• das Verarbeitungsmittel (102) eine oder mehrere neue Modifikationen bestimmt, die an der Aufstellung der Füße vorgenommen werden müssen, um eine ideale Haltungsaufstellung des/der vordefinierten Körperteile(s) des/der Patienten/Patientin zu erreichen.

4. System nach Anspruch 3, wobei das System (100) so ausgelegt ist, dass es nicht mehr zur Durchführung weiterer Iterationen betrieben wird, wenn das Verarbeitungsmittel (102) feststellt, dass die ideale Haltungsaufstellung von dem/den vordefinierten Körperteil(en) des/der Patienten/Patientin erreicht wurde.

5. System nach Anspruch 4, wobei das Verarbeitungsmittel (102) auch so programmiert ist, dass es, sobald die ideale Haltungsaufstellung erreicht wird, auf der Grundlage der idealen Haltungsaufstellung Daten/Informationen zur Herstellung von Einlegesohlen erzeugt, die es dem/den vordefinierten Körperteil(en) des/der Patienten/Patientin ermöglichen, die ideale Haltungsaufstellung einzunehmen, wenn sie vom/von der Patienten/Patientin benutzt wird/werden.

6. System nach Anspruch 5, wobei das Verarbeitungsmittel (102) so konfiguriert ist, dass es eine oder mehrere vordefinierte Technologien der künstlichen Intelligenz implementiert, um:
• die Verarbeitungs- und Analysefunktion auszuführen;
• festzustellen, dass die ideale Haltungsaufstellung erreicht wurde;
• die Daten/Informationen zur Herstellung der Einlegesohlen zu erzeugen.

7. System nach Anspruch 6, wobei die vordefinierte(n) künstliche(n) Intelligenztechnologie(n) eine oder mehrere maschinelle Lerntechniken und/oder ein oder mehrere neuronale Netze einschließt/einschließen.

8. Verfahren zur Herstellung optimierter Einlegesohlen, umfassend die Verwendung des Systems (100) nach einem der vorhergehenden Ansprüche, um Einlegesohlen für eine(n) Patienten/in herzustellen, so dass diese(r) eine ideale Haltungsaufstellung einnehmen kann.

## Revendications

1. Système (100) pour la fabrication de semelles optimisées, comprenant des moyens de détection (101) et des moyens de traitement (102) ;
dans lequel les moyens de détection (101) sont configurés pour exécuter une fonction de détection qui inclut :
- les quantités de détection relatives à
- une configuration posturale et des schémas de position et de mouvement d'une ou de plusieurs parties prédéfinies du corps d'un patient en orthostase et pendant la marche, et
- une disposition des différents éléments des pieds du patient en orthostase et pendant la marche ; et
- générer, sur la base des quantités détectées, des données de détection correspondantes ;
dans lequel les moyens de traitement (102) sont configurés pour acquérir à partir des moyens de détection (101) les données de détection générées par lesdits moyens de détection (101) et sont programmés pour exécuter une fonction de traitement et d'analyse incluant :
- effectuer un traitement prédéfini des données de détection acquises en déterminant, sur la base du traitement prédéfini effectué,
- la configuration posturale et la position et les schémas de mouvement adoptés par la ou les parties prédéfinies du corps du patient en orthostase et pendant la marche, et
- la disposition des différents éléments des pieds du patient pendant que ce dernier adopte lesdits modèles de position et de mouvement ;
- analyser la configuration posturale et les schémas de position et de mouvement déterminés pour détecter un ou plusieurs schémas pathologiques et un ou plusieurs déséquilibres posturaux correspondants de ladite ou desdites parties prédéfinies du corps du patient ; et
- déterminer, sur la base du ou des schémas pathologiques et du ou des déséquilibres posturaux correspondants détectés, un ou plusieurs changements correspondants à apporter à la configuration des pieds et des membres inférieurs du patient, de manière à permettre de modifier la configuration posturale et/ou les schémas de position et de mouvement adoptés par ladite ou lesdites parties prédéfinies du corps du patient, afin de ne plus adopter ledit ou lesdits schémas pathologiques grâce à la correction dudit ou desdits déséquilibres posturaux correspondants.

2. Système de la revendication 1, dans lequel les moyens de traitement (102) sont programmés pour exécuter la fonction de traitement et d'analyse par :
- déterminer, sur la base des données de détection acquises, des modèles numériques multidimensionnels relatifs à ladite configuration posturale et aux modèles de position et de mouvement adoptés par la ou les parties prédéfinies du corps du patient en orthostase et pendant la marche ; et
- détecter le(s) schéma(s) pathologique(s) et le(s) déséquilibre(s) postural(aux) correspondant(s) et déterminer la(les) modification(s) correspondante(s) à apporter sur la base des modèles numériques multidimensionnels déterminés.

3. Système selon la revendication 1 ou 2, dans lequel ledit système (100) est conçu pour être exploité de manière itérative de sorte qu'à chaque itération :
- le moyen de détection (101) et le moyen de traitement (102) exécutent respectivement la fonction de détection et la fonction de traitement et d'analyse après que la ou les modifications déterminées par le moyen de traitement (102) à l'itération immédiatement précédente ont été apportées à la configuration des pieds du patient afin de déterminer une modification conséquente de la configuration posturale et de la position et des schémas de mouvement de la ou des parties prédéfinies du corps du patient en orthostase et pendant la marche ; et
- le moyen de traitement (102) détermine une ou plusieurs nouvelles modifications à apporter à la configuration des pieds afin d'obtenir une configuration posturale idéale de la ou des parties prédéfinies du corps du patient.

4. Système de la revendication 3, dans lequel ledit système (100) est conçu pour ne plus être utilisé pour effectuer d'autres itérations lorsque les moyens de traitement (102) déterminent que la configuration posturale idéale a été atteinte par la (les) partie(s) prédéfinie(s) du corps du patient.

5. Système de la revendication 4, dans lequel le moyen de traitement (102) est également programmé pour, une fois que la configuration posturale idéale a été atteinte, générer, sur la base de cette configuration posturale idéale, des données/informations pour la fabrication de semelles permettant, lorsqu'elles sont utilisées par le patient, à la (aux) partie(s) prédéfinie(s) du corps du patient d'adopter ladite configuration posturale idéale.

6. Système de la revendication 5, dans lequel le moyen de traitement (102) est configuré pour mettre en œuvre une ou plusieurs technologies d'intelligence artificielle prédéfinies pour :
- assurer la fonction de traitement et d'analyse ;
- déterminer que la configuration posturale idéale a été atteinte ;
- générer les données/informations nécessaires à la fabrication des semelles.

7. Système de la revendication 6, dans lequel la ou les technologies d'intelligence artificielle prédéfinies incluent une ou plusieurs techniques d'apprentissage automatique et/ou un ou plusieurs réseaux neuronaux.

8. Procédé de fabrication de semelles optimisées, comprenant l'utilisation du système (100) selon l'une quelconque des revendications précédentes pour fabriquer des semelles pour un patient de manière à permettre à ce dernier d'adopter une configuration posturale idéale.
